(19) Europäisches Patentamt
European Patent Office
Office européen des brevets

(11) Numéro de publication: **0 184 480**
**A1**

(12) # DEMANDE DE BREVET EUROPEEN

(21) Numéro de dépôt: 85402157.3

(22) Date de dépôt: 07.11.85

(51) Int. Cl.⁴: C 08 B 37/14
A 61 K 31/72

(30) Priorité: 07.11.84 FR 8417460

(43) Date de publication de la demande:
11.06.86 Bulletin 86/24

(84) Etats contractants désignés:
AT BE CH DE FR GB IT LI LU NL SE

(71) Demandeur: SANOFI
40, Avenue George V
F-75008 Paris(FR)

(71) Demandeur: SCLAVO S.p.A.
Via Fiorentina 1
I-53100 Siena(IT)

(72) Inventeur: Bayol, Alain
5 rue de la Sardane
F-31170 Tournefeuille(FR)

(72) Inventeur: Blanc, Francis
Chemin de Soriech
F-34000 Lattes(FR)

(72) Inventeur: Lansen, Jacqueline
120, rue des Chardonnerets Saint Clément
F-34980 St Gely du Fesc(FR)

(72) Inventeur: Maffrand, Jean-Pierre
5 rue du Corps-Franc Pommiès
F-31120 Portet/Garonne(FR)

(72) Inventeur: Pereillo, Jean-Marie
9 avenue des Mimosas
F-31120 Portet/Garonne(FR)

(74) Mandataire: Bressand, Georges et al,
c/o CABINET LAVOIX 2 Place d'Estienne d'Orves
F-75441 Paris Cedex 09(FR)

(54) **Nouveaux sulfates de xylanes de bas poids moléculaires, leur procédé de préparation et médicaments les contenant.**

(57) La présente invention est relative à de nouveaux sulfates de xylanes de masse moléculaire moyenne comprise entre 2 000 et 5 000 Daltons.

L'invention est également relative à leur procédé de préparation par fractionnement et à leur application en thérapeutique en tant qu'agents antithrombotique et hypolipémiant actifs par voie orale.

EP 0 184 480 A1

Croydon Printing Company Ltd

La présente invention est relative à de nouveaux sulfates de xylanes, à leur procédé de préparation et à leur application en médecine humaine et vétérinaire pour leur activité antithrombotique et hypolipémiante.

Dans ce domaine d'activité, l'héparine est un mucopolysaccharide d'origine animale largement utilisé en thérapeutique humaine, notamment dans la prévention et le traitement des thromboses veineuses et des embolies pulmonaires.

Les héparines commerciales sont constituées en fait d'un mélange de polysaccharides dont les masses molaires varient entre 3000 et 50000 Daltons. Environ 80 % de ces polymères ont une masse molaire comprise entre 5000 et 25000 D et la masse molaire moyenne du mélange se situe autour de 16000 D.

Ces polymères, en raison de leur masse molaire élevée présentent une biodisponibilité quasi-nulle par voie orale et, de ce fait, les héparines standards ne sont efficaces qu'après administration parentérale.

Plus récemment, diverses équipes ont fractionné ou fragmenté ces héparines standards pour obtenir des héparines de bas poids moléculaires (masse molaire moyenne comprise entre 4000 et 8000 D) qui semblent présenter moins de risques hemorragiques et thrombocytopémiants que les héparines standards.

D'autres polysaccharides sulfatés ont présenté, en pharmacologie animale et en clinique humaine, des propriétés antithrombotiques comparables à celles de l'héparine. L'un de ces produits, appelé SP 54 ou PZ 68 selon les auteurs est un polysulfate de xylane obtenu par sulfatation ménagée de xylanes extraits de bois de Hêtre, à un coût très inférieur à celui de l'héparine.

Ce produit est commercialisé depuis une vingtaine d'années pour utilisation par voie injectable, notamment dans la prévention des thromboses veineuses post-opératoires, et par voie orale, notamment dans le traitement des dyslipémies athérogènes.

Par ses effets sur les systèmes de coagulation (FISCHER A.M., BARROWCLIFFE T.W.&THOMAS D.P. Thromb Haemost, 1982, 47, (2), 104) (FISCHER A.M., MERTON R.E., MARSH N.A., WILLIAMS S., GAFFNEY P.J., BARROWCLIFFE T.W. & THOMAS D.P. Thromb Haemost, 1982, 47, (2), 109) (SCULLY M.F., WEERASINGHE K.M., ELLIS V., DJAZAERI B. & KAKKAR V.V. Thromb Res, 1983, 31, 87) et de fibrinolyse (VINAZZER H., STEMBERGER A., HAAS S. & BLÜMEL G., Thromb Res, 1982, 27, 341), sur le système complémentaire (WALB D., LOOS M. & HADDING U. Z Natusforsch, 1971, 26, 403), sur la paroi des vaisseaux (PAUL R., HERBERT J.M., MAFFRAND J.P., LANSEN J. & RONCUCCI R., Symposium on Atheroma and Thrombosis, London, July 1983, et sur le métabolisme lipidique (ROUFFY J., Mises à jour Cardiol, 1983, 12 (8), 381, ce produit présente en effet des propriétés antithrombotique et antiathéromateuse intéressantes.

Comme l'héparine, il se présente sous la forme d'un mélange de polymères (sulfates de xylanes) de différents poids moléculaires. La définition analytique de tels mélanges est toujours délicate et plusieurs expérimentateurs ont pu donner des résultats différents pour un même produit.

Ainsi, VINAZZER et al. ont reporté le SP 54 un poids moléculaire de 2000 (Thromb. Res., 1980, 20, 57-68) puis de 3000 (Thromb. Res., 1982, 27, 341-352) ; C.D. ESQUIVEL et al. (Thromb. Res., 1982, 28 ,389-399) ont décrit un poids moléculaire moyen de 4000 ; enfin C. SORIA et al. (Thromb. Res., 1980, 19, 455-463) ont signalé un poids moléculaire moyen de 6000 qui a été aussi publié très récemment par M.F. SCULLY et V.V. KAKKAR (Biochem. J., 1984, 218, 657).

Cette disparité dans les poids moléculaires moyens, est sans doute due aux méthodes d'analyse et aux standards utilisés ainsi qu'à l'absence de précision sur la nature (apparente, en poids, en nombre) de la masse molaire publiée.

A l'aide des techniques décrites plus loin, la demanderesse a trouvé que le SP 54 ou PZ 68 qui répond à la formule statistique développée suivante :

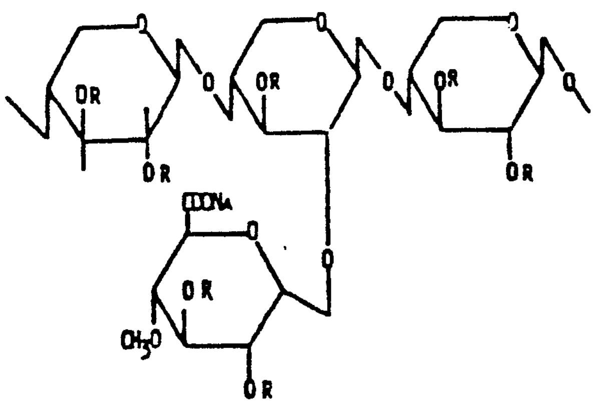

I

dans laquelle R représente le groupe $-SO_3$ Na ou l'hydrogène peut être défini de la manière suivante :

Il s'agit d'un mélange de polymères de masses molaires comprises entre 1000 et 40000 Daltons, présentant une distribution quasi-gaussienne.

Les mesures de paramètres de distributions moléculaires de ce produit par chromatographie d'exclusion haute performance font apparaitre pour différents lots de fabrication des valeurs moyennes de :

Masse moléculaire moyenne en nombre ou $\overline{Mn}$ : 5300 à 5500

Masse moléculaire moyenne en poids ou $\overline{Mw}$ : 7400 à 7600

Indice de polydispersité ou $D = \frac{\overline{Mw}}{\overline{Mn}}$ mesurant la largeur de la distribution moléculaire : 1,4 à 1,5.

La masse moyenne apparente ou $\overline{Ma}$ qui correspond à la masse moléculaire de la population isomoléculaire majoritaire en concentration (c'est-à-dire le sommet du pic sur le chromatogramme) est comprise entre 6 500 et 7 200 D.

Le taux de sulfatation du produit c'est-à-dire le nombre de fonctions hydroxyle estérifiées par un groupement sulfurique est accessible par le dosage pondéral moyen du soufre : 15 à 20 %.

On peut définir aussi le degré de sulfatation moyen de ce mélange de polymères ou D.S. comme le nombre moyen de groupes sulfuriques par unité xylose considéré comme élément monomère.

Le degré de sulfatation moyen pour le produit, accessible par résonance magnétique nucléaire du proton ou par dosages centésimaux microanalytiques du carbone et du soufre est compris entre 1,5 et 2 et plus précisément proche de 1,8.

Ce polysulfate de xylane est aussi caractérisé par les valeurs moyennes des dosages de pentoses et d'acides glucuroniques.

Ainsi le pourcentage pondéral de pentoses exprimé en équivalent xylose est de 30 à 40 %.

Le pourcentage pondéral d'acides uroniques (en fait principalement acide 4 MeO-glucuronique) exprimé en acide glucuronique varie de 4 à 6 %.

Ceci correspond à une proportion de 1 unité glucuronique pour 8 à 10 unités xyloses.

La Demanderesse a maintenant trouvé que de nouveaux sulfates de xylane, ayant un faible poids moléculaire et des caractéristiques physicochimiques bien déterminées, possèdent une bonne activité par voie orale, avec une excellente absorption duodénale.

Ainsi, la présente invention a pour objet des polysulfates de xylane ayant les caractéristiques suivantes :

- degré de sulfatation moyen est compris entre 1,5 et 2
- masse moléculaires moyennes apparentes $\overline{Ma}$ comprises entre 2000 et 5000 Daltons.
- taux pondéral de pentoses exprimés en xylose compris entre 30 et 40 %
- taux pondéral d'acides uroniques exprimé en acide glucuronique compris entre 0 et 10 %
- les indices de polydispersité des distributions moléculaires pour les produits de l'invention sont inférieures ou égales à celles du produit . Ces produits ne contiennent que des quantités négligeables de polymères de masse unitaire supérieure à 10000 Daltons.

Ces sulfates de xylanes de bas poids moléculaire présentent une biodisponibilité par voie orale nettement supérieure à celle du SP 54 et leur absorption duodénale est plus importante que l'absorption gastrique. Toutes ces observations ont conduit à la mise au point de formes galéniques permettant une bonne absorption par voie orale, chez l'homme.

La demanderesse a enfin trouvé que ces nouveaux produits de la présente invention, présentaient, d'une façon surprenante une activité pro-agrégante vis à vis de l'ADP nettement inférieure à celle du SP 54 et surtout de l'Héparine, laissant ainsi espérer moins de risques thrombocytopémiants qu'avec ces produits.

Ces nouveaux polysulfates de xylane peuvent être préparés par un procédé caractérisé en ce que le produit SP 54 est directement soumis à un fractionnement par ultra-filtration sur des membranes ou des fibres creuses de différentes porosités.

Les caractéristiques analytiques du SP 54 décrites plus haut et celles des produits finaux ont été déterminées selon les méthodes ci-après :

1 - Spectre de résonnance magnétique nucléaire du carbone 13

Le spectre de résonnance magnétique nucléaire du carbone 13 (80 MHz) est réalisé sur une solution à 10 % (m/V) dans l'eau deutériée. Les déplacements chimiques des pics correspondant aux atomes de carbone de l'unité xylose sulfatée, sont les suivants : $C_1$ : 100,4 ppm ; $C_2$ : 73,6 ppm ; $C_3$ : 74,1 ppm ; $C_4$ : 75,43 ppm et $C_5$ : 60,3 ppm.

2 - Spectre de résonance magnétique nucléaire du proton

Le spectre de résonnance magnétique nucléaire du proton (250 $MH_2$) est réalisé à la concentration de 20 % (m/V) dans l'eau deutériée. Le signal correspondant à l'eau est déplacé par ajout d'acide trifluoroacétique. Le degré de sulfatation peut être calculé sur la base de l'intégration des protons 1 et 3 (5,2 ppm et 4,8 ppm) de l'unité xylose sulfatée.

3 - Dosage des groupements méthoxy

La méthode employée est celle décrite par LAVER M. et WOLFROM M.L. dans Methods in Carbohydrate Chemistry Vol. I, page 454, Edité par Ac. Press. 1962.

4 - Dosage des acides uroniques

La méthode employée est celle au carbazole de BITTER T. et MUIR H.M. (Anal Biochem, 1962, 4, 330-334).

5 - Microdosage du soufre

Après minéralisation en fiole de SHONIGER W. (Mikrochim Acta 1956, 1, 869-876), le soufre est dosé conductrimétriquement par le perchlorate de baryum.

Les pourcentages centésimaux de soufre et de carbone (obtenu par microanalyse) permettent de calculer le degré de sulfatation ou D.S. :

- nombre de moles de soufre pour 100 g de Produit : $\frac{\% S}{32,06}$

- nombre de moles de carbone pour 100 g de Produit : $\frac{\% C}{12,011}$
- nombre de moles de xylose pour 100 g de Produit : $\frac{\% C}{12,011 \times 5}$
- nombre moyen de fonctions sulfates par xylose monomère ou

$$D.S. = 5 \times \frac{\% S}{\% C} \times \frac{12,011}{32,06}$$

6 - Dosage des pentoses

La méthode employée est celle à l'orcinol et au chlorure ferrique en milieu chlorhydrique (MEJBAUM W. - Z.Physiol. Chem. 1979, 258, 117) en prenant le xylose comme étalon.

7 - Dosage du sulfate de sodium

Il est réalisé par chromatographie liquide haute performance en utilisant une colonne IONOSPHER TmA de 25 cm de longueur (CHROMPACK ref. 28300) avec comme éluant de l'hydrogène phtalate de potassium à 0,7 % (m/V) dans l'eau distillée. L'étalonnage est réalisé avec du sulfate de sodium anhydre.

8 - Détermination de la masse moléculaire

Elle est réalisée par chromatographie d'exclusion à travers deux colonnes de silice de porosité 60 Å et 500 Å placées en série. L'appareil utilisé est celui de HEWLETT PACKARD 1081 B équipé d'un réfractomètre JOBIN et YVON IOTA et d'un injecteur HP 79841 A. L'ensemble est thermostaté à 30 °C. La phase mobile contenant du sulfate de sodium est préparée selon BARTH H.G. et SMITH D.A. (J. Chromatogr. 1981, 206, 410-415) et modifié en ajoutant 0,5 % de polyéthylène glycol pour éviter les interactions de type pont hydrogène. La force ionique de l'éluant est égale à 1.
Les masses moléculaires moyennes en nombre ($\overline{Mn}$), en poids ($\overline{Mw}$) et apparente ($\overline{Ma}$) sont calculées à partir d'un étalonnage réalisé à l'aide d'oligosaccharides neutres (xylose, maltotriose) et de Dextranes (T40-T70 et T500) selon la méthode décrite par YAU W.W. ; KIRKLAND J.J. et BLY D.D. dans Modern Size-exclusion Liquid Chromatography page 315 Edité par J. WILEY et SONS (1979).

L'indice de polydispersité est calculé selon le rapport $\overline{Mw}/\overline{Mn}$.

Les exemples non limitatifs suivants illustrent l'invention :

Exemple 1: Fraction de polysulfate de xylan ou SP 54 de masse apparente 3346 Daltons.

Une solution de 1 800 g de polysulfate de xylane dans 24 litres d'eau déminéralisée (concentration poids volume 7,5 %) est ultrafiltrée dans un système équipé d'une cartouche de fibres creuses ROMICON 22-20 PM 5 (Longueur 63 cm surface de membrane 2,4 m², seuil de coupure 5 000) , et d'une admission d'eau déminéralisée permettant d'ultrafiltrer la solution mère en conservant son volume constant.

La pression moyenne de travail en amont de la cartouche est réglée à 1,5 Bar et le débit moyen d'ultrafiltrat obtenu est de l'ordre de 130 ml/mn. Lorsque 60 litres de perméat ont été soutirés du système, la solution mère retenue est retirée du système que l'on rince alors à l'eau déminéralisée. On remplace la cartouche utilisée par une ROMICON 15-43 PM 2 (Longueur 63 cm, surface de membrane 1,4 m², seuil de coupure 2000).

On concentre alors la solution d'ultrafiltrat obtenue précédemment sous une pression de 1,5 Bar jusqu'à un volume de 19 litres.

Les 41 litres de solution de perméat de concentration recueillis hors du système sont alors lyophilisés dans un appareil industriel.

On recueille 189 g de cristaux blancs (10,5 % de rendement par rapport à la quantité initiale de sulfate de xylane).

Ces cristaux, contenant les fractions de plus petits poids moléculaire du produit initial et ses impuretés minérales (principalement du sulfate de sodium) qui s'y trouvent ainsi concentrées, sont alors dessalés par quantités de 3 g sur une colonne de gel Trisacryl GF 05 (Longueur 90 cm, diamètre 44 mm, débit d'eau distillée 3 ml/mm, détection réfractométrique).

Les fractions d'éluant de colonne correspondant aux petits poids moléculaires de polysulfate de xylane séparées des parties d'éluat contenant les impuretés minérales sont alors lyophilisées.

En 10 opérations identiques de dessalage, on recueille : 14,2 g de sulfate de xylane de petits poids moléculaires exempts de sulfate de sodium.

Rendement par rapport à la quantité initiale de sulfate de xylane : 5 %.

Caractéristiques analytiques

Distribution moléculaire

$\overline{Mn}$ = 2794 $\overline{Mw}$ = 3380 D = 1,21 $\overline{Ma}$ : 3346

Composition chimique

- Degré de sulfatation 1,88 par microanalyse Soufre/Carbone
- Pourcentage de Pentoses exprimés en xylose : 31,4 %
- Pourcentage d'acide uronique exprimés en acides glucuroniques : 6 %
- Teneur en sulfate de sodium : < 0,3 %

Exemple 2 : Fraction de polysulfate de xylane de poids moléculaire moyen 3518 Daltons.

Une solution de 1,5 kg de polysulfate de xylane dans 60 litres d'eau permutée, est ultrafiltrée dans un système tel que celui décrit dans l'exemple 1 équipé d'une membrane ROMICON 15-43 PM 2 (Longueur 63 cm, surface de membrane 1,4 m², seuil de coupure 2000) ; sous 1 Bar de pression, à volume constant.

Lorsque 60 litres d'ultrafiltrat ont été recueillis

l'opération est stoppée et le système est nettoyé pour une opération identique.

Les solutions perméat de trois opérations successives soit 180 litres obtenues de 3 fois 1,5 Kg de polysulfate de xylane sont alors concentrées à 45 litres sur le même système équipé cette fois d'un modèle spiral millipore PCAC 1 000, de seuil de coupure 1 000 en acétate de cellulose.

Losque le volume de solution rétentat de 45 litres a été obtenue (135 litres d'ultrafiltrat de concentration soutirés du système), l'admission d'eau permutée est ouverte pour opérer une ultrafiltration à volume constant. On soutire ainsi : 70 litres de solution d'ultrafiltrat. Puis la solution de rétentat est soutirée du système et concentrée dans un évaporateur sous vide jusqu'à un volume de 7 litres. On opère alors une lyophilisation qui permet d'isoler 402 g de cristaux blancs.

Rendement par rapport à la quantité initiale de polysulfate de xylane (4,5 kg) : 9 %.

Caractéristiques analytiques
----------------------------

Distribution moléculaire :

$\overline{Mn}$ = 2732 $\overline{Mw}$ = 4183 D = 1,53 $\overline{Ma}$ = 3518

Composition chimique

- Degré de sulfatation : 1,8 par RMN
- Pourcentage de pentoses exprimé en xylose : 30 %
- Pourcentage d'acides uroniques exprimé en acide glucuronique : 4,3 %
- Teneur en sulfate de sodium : 2,2 %.

Exemple 3 : Fraction de polysulfate de xylane de poids moléculaire moyen apparent 3889 Daltons.

Une solution de 60 g de polysulfate de xylane dans 1 000 ml d'eau est ultrafiltrée sur fibres creuses AMICON H1P 10-8 dans un système, à volume de rétentat constant.

On recueille 1100 ml d'ultrafiltrat que l'on concentre à 800 ml, sur le même système équipé de fibres creuses HIP

5-20 (seuil de coupure 5000), puis on ultrafiltre à volume constant en utilisant 4 000 ml d'eau distillée. Les 4300 ml d'ultrafiltrat recueillis lors de cette dernière opération sont concentrés à 800 ml sur le même système équipé d'un faisceau de fibres creuses HIP-2-43 (seuil de coupure 2000), puis l'on ultrafiltre à volume de rétentat constant en utilisant 1600 ml d'eau distillée. Le rétentat est alors soutiré du système, et lyophilisé. On recueille 11,3 g de poudre blanche (19 %). de rendement.

Caractéristiques analytiques

Distribution moléculaire

$\overline{Mn}$ : 3330  $\overline{Mw}$ : 4480  D = 1,34  $\overline{Ma}$ = 3889

Composition chimique :

- Degré de sulfatation : 1,93 par microanalyse carbone soufre
- Pourcentage de pentoses exprimé en xylose : 38 %
- Pourcentage d'acides uroniques exprimé en acide glucuronique : 4,9 %

Exemple 4 : Fraction de polysulfate de xylane de poids moléculaire 3939 Daltons.

Une solution de 10 g de polysulfate de xylane dans 500 ml d'eau distillée (concentration poids/volume 2 %) est ultrafiltrée à volume constant sur un système équipé d'une cartouche de fibres creuses AMICON H1P-2-43 de seuil de coupure 2 000.

Lorsque 1250 ml d'ultrafiltrat ont été recueillis l'opération est arrêtée.

La teneur en sulfate de sodium de cette solution est ramenée à 1 % par concentration et ultrafiltration à volume constant sur un système équipé de membranes planes AMICON 5 YM 2 de seuil de coupure 1 000.

On lyophilise la solution finale et on obtient 1,2 g de cristaux blancs (Rendement : 12 %).

Caractéristiques analytiques

Distribution moléculaire

$\overline{Mn}$ = 3007 $\overline{Mw}$ = 4013 D = 1,33 $\overline{Ma}$ = 5939

Composition chimique

- Degré de sulfatation : 1,85 par RMN
- Pourcentage de pentoses exprimé en xylose : 34,3 %
- Pourcentage d'acides uroniques exprimé en acide glucuronique : 5,1 %
- Teneur en sulfate de sodium : 1,8 %

Exemple 5 : Fraction de polysulfate de xylane de poids moléculaire moyen 4066 Daltons.

Une solution de 1 kg de polysulfate de xylane dans 33 litres d'eau permutée (concentration poids/volume 3 %) est ultrafiltrée dans un système équipé d'une cartouche de fibres creuses ROMICON 15-43 PM 2 (Longueur 63 cm, surface de membrane 1,4 m², seuil de coupure 2000) et permettant de travailler à volume de rétentat constant sous une pression de 1,2 Bar.

Lorsque 50 litres d'ultrafiltrat ont été obtenus la solution de rétentat est soutirée.

Le système est équipé d'un module spiral Millipore PCAC 1000 (acétate de cellulose, seuil de coupure 1000) pour traiter la solution d'ultrafiltrat obtenue précédemment. Cette solution est concentrée à 42 litres sous une pression de 0,8 Bar, puis l'admission d'eau permettant de garder le volume de solution traitée constant est ouverte.

Lorsque 63 litres d'ultrafiltrat ont été recueillis, la solution rétentat est soutirée du système, son volume est ramené de 42 litres à 8 litres dans un concentrateur sous vide, puis lyophilisée.

On obtient 109 g de cristaux blancs (Rendement : 11 %).

Caractéristiques analytiques

Distribution moléculaire

$\overline{Mn}$ = 3133 $\overline{Mw}$ = 4716 D = 1,50 $\overline{Ma}$ = 4066

Composition chimique

- Degré de sulfatation : 1,82 par RMN

- Pourcentage de pentoses exprimé en xylose : 34,5 %
- Pourcentage d'acides uroniques exprimé en acide glucuronique : 4,3 %
- Teneur en sulfate de sodium : 0,3 %

Exemple 6 : Fraction de polysulfate de poids moléculaire moyen 4407 Daltons

Une solution de 30 g de polysulfate de xylane (SP 54) dans 1 000 ml d'eau distillée est ultrafiltrée dans un système équipé d'un faisceau de fibres creuses H1 P 5-20, à volume de rétentat constant en utilisant 5 000 ml d'eau distillée en apport continu dans le rétentat. On recueille 5 000 ml d'ultrafiltrat que l'on concentre à l'aide du même système équipé de fibres H1P 2-43, à 1 000 ml. On ultrafiltre ensuite à volume de rétentat constant avec apport continu d'eau distillée (5 000 ml). Le rétentat est alors retiré du système et lyophilisé.

On recueille 9 g de poudre blanche (30 % de rendement).

Caractéristiques analytiques

Distribution moléculaire

$\overline{Mn}$ = 3470    $\overline{Mw}$ = 4570    D = 1,3    $\overline{Ma}$ = 4407

Composition chimique

- Degré de sulfatation : 1,90 par microanalyse carbone soufre
- Pourcentage d'acides uroniques exprimé en acide glucoronique : 4,7 %
- Teneur en sulfate de sodium : 1,6 %

Exemple 7: Fraction de polysulfate de xylane de poids moléculaire moyen 4674 Daltons

Une solution de 10 g de polysulfate de xylane dans 500 ml d'eau distillée (concentration poids/volume 2 %) est ultrafiltrée dans un système permettant de travailler à volume de rétentat constant, équipé d'une cartouche de fibres creuses AMICON H1P 3-20 (seuil de coupure 3 000 daltons, sous une pression de 0,9 Bars).

Lorsque 1250 ml d'ultrafiltrat ont été soutirés du système, cette dernière solution est concentrée sur un système

équipé d'une membrane plane Millipore PCAC 1000 (seuil de coupure 1000 Daltons) jusqu'à un volume de 400 ml puis ultrafiltrée à volume constant.

Lorsque 800 ml d'ultrafiltrat ont été recueillis, la solution rétentat est lyophilisée.

On recueille 2,7 g de cristaux blancs, rendement 27 %.

Caractéristiques analytiques

Distribution moléculaire

$\overline{Mn}$ = 3648 $\overline{Mw}$ = 4958 D = 1,36 $\overline{Ma}$ = 4674

Composition chimique

- Degré de sulfatation : 1,80 par RMN
- Pourcentage de pentoses exprimé en xylose : 35,2 %
- Pourcentage d'acides uroniques exprimé en acide glucuronique : 5,3 %
- Teneur en sulfate de sodium : 2,1 %

Exemple 8 : Fraction de polysulfate de xylane de poids moléculaire moyen 4903 Daltons

Une solution de 30 g de polysulfate de xylane dans 450 ml d'eau distillée (concentration poids/volume 6,66 %) est ultrafiltrée à volume de rétentat constant dans un système équipé d'une cartouche de fibres creuses AMICON H1P 5-20 (seuil de coupure 5 000 Daltons) sous une pression de 0,9 Bar. On recueille 1200 ml d'ultrafiltrat que l'on retraite sur un système équipé d'une membrane plane Millipore PCAC 1000.

Après concentration à 400 ml, on ultrafiltre à volume constant. Après avoir receuilli 600 ml d'ultrafiltrat, le rétentat est lyophilisé.

On obtient 8,5 g de cristaux blancs (Rendement 28 %)

Caractéristiques analytiques

Distribution moléculaire

$\overline{Mn}$ = 4039 $\overline{Mw}$ = 5058 D = 1,25 $\overline{Ma}$ = 4903

Composition chimique

- Degré de sulfatation : 1,98 par microanalyse carbone-soufre

- Pourcentage de pentoses exprimé en xylose : 30,8 %
- Pourcentage d'acides uroniques exprimé en acide glucuronique : 4,6 %
- Teneur en sulfate de sodium : 2,3 %.

Exemple 9 : Fraction de polysulfate de xylane de poids moléculaire moyen 4921 Daltons.

Une solution de 2,5 kg de polysulfate de xylane dans 50 litres d'eau permutée (concentration poids volume 2 %) est ultrafiltrée dans un système équipé d'une cartouche ROMICON 15-43 PM2 (Longueur 63 cm, surface de membrane : 1,4 $m^2$, seuil de coupure 2000 Daltons), et d'un système d'admission d'eau permutée permettant de travailler à volume de rétentat constant, sous une pression de 850 mBars.

On soutire ainsi du système 31 litres d'ultrafiltrat que l'on dessale ensuite à volume constant, sous une pression de 1,2 Bar sur un système équipé d'un modèle spiral Millipore PCAC 1000 (acétate de cellulose seuil de coupure 1000 Daltons). Après avoir éliminé 49,5 litres d'ultrafiltrat, la solution rétentat est concentrée sous vide jusqu'à un volume de 9,2 litres, puis lyophilisée. On recueille 658 g (rendement 26 %) de cristaux blancs.

Caractéristiques analytiques

Distribution moléculaire

$\overline{Mn}$ = 3680   $\overline{Mw}$ = 5585   D = 1,52   $\overline{Ma}$ = 4921

Composition chimique

- Degré de sulfatation : 2,0 par RMN
- Pourcentage de pentoses exprimé en xylose : 34,7 %
- Pourcentage d'acides uroniques exprimé en acide glucuronique : 5,7 %
- Teneur en sulfate de sodium : 2,3 %

Les résultats des essais toxicologiques et pharmacologiques ont permis de mettre en évidence les intéressantes propriétés des produits de l'invention. C'est-à-dire des différentes fractions qui ont été obtenues à partir du SP 54.

L'invention a donc pour objet un médicament ayant en particulier des propriétés anti-thrombotiques et hypolipémiantes, caractérisé en ce qu'il contient, à titre de principe actif, au moins une des fractions préparées par le procédé de l'invention et répondant à la formule (I).

L'étude toxicologique a montré la faible toxicité et la parfaite tolérance des différentes fractions de l'invention. En effet, les essais effectués sur la toxicité aiguë, chronique, subchronique et retardée, chez différentes espèces animales, n'ont mis en évidence aucune anomalie d'aucune sorte. L'étude pharmacologique a porté sur les actions anticoagulante, fibrinolytique et lipolytique par les voies orale et intraduodénale et sur l'activité proagrégante plaquettaire vis-à-vis de l'ADP, in vitro ; ces études ont été effectuées comparativement au SP 54.

- L'action anticoagulante est déterminée par le temps de céphaline activée (CAEN J., LARRIEU M.J., SAMAMA M., In : L'hémostase : méthode d'exploration et diagnostic pratique. Ed. : L'Expansion Scientifique Française, 1975, p. 169).
  Le temps de céphaline activée (TCA), mesure la recalcification du plasma déplaquetté en présence d'un optimum de lipides (Céphaline) et de célite qui active de manière standardisée les facteurs de la phase de contact (les facteurs XII, XI, IX). Il mesure donc la formation de prothrombinase endogène à l'exception des facteurs plaquettaires remplacés par la céphaline.
- L'activité fibrinolytique est mesurée par le temps de lyse des euglobulines plasmatiques (KLUFT C.- Haemostasis, 1976, 5, 136).
  Le temps de lyse des euglobulines plasmatiques est une méthode sensibilisée permettant d'apprécier l'action fibrinolytique globale du SP 54 et de ses fractions en l'absence des inhibiteurs physiologiques de la fibrinolyse, éliminés par dilution et acidification du plasma.
- L'action lipolytique est mesurée par l'activité lipoprotéine lipase circulante (NIKKILA E.A., HUTTUNEN J.K., EHNHOLM C., - Metabolism, 1977, 26, 179).
La détermination de l'activité lipoprotéine lipase circulante est une mesure indirecte de l'impact du SP 54 et de ses fractions sur le catabolisme des triglycérides (pouvoir clarifiant). L'activité enzymatique est déterminée par sa capacité d'hydrolyser le substrat 14C-trioléine en 14C-acide oléique.

- Activité proagrégante plaquettaire
L'activité proagrégante des sulfates de xylane a été évaluée par leur capacité éventuelle de potentialiser de manière irréversible l'agrégation plaquettaire réversible induite par de faibles concentrations d'ADP.
Ces études ont été effectuées selon la technique de Born sur du PRP d'origine humaine. Les divers produits à tester sont incubés pendant 1 mn à 37 °C en présence de PRP. Des concentrations croissantes de 2 à 125 µg/ml sont utilisées; Après cette période d'incubation, l'agrégation plaquettaire a été induit par de faibles concentrations d'ADP (0,5 à 2 µM) provoquant du moins pour les contrôles un phénomène réversible d'agrégation.

Les résultats sont exprimés en concentration minimale de sulfate de xylane permettant l'obtention d'un phénomène irréversible d'agrégation.
Référence : G.U.R. Born and J. Cross : The aggregation of blood platelets J. Physiol., 168, 178, 186).

- Administration intraduodénale et orale des rats mâles (Sprague Dawley ; Charles River ; France) de 200 à 300 g sont utilisés. Les animaux sont soumis à une diète hydrique la veille des essais.
Une anesthésie au pentobarbital (50 mg/kg : i.p.) est effectuée sur tous les animaux avant l'administration des différentes fractions de sulfates de xylane. Ceux-ci sont administrés à une dose unique de 400 mg/kg.
Pour l'administration intraduodénale, une incision est effectuée au niveau de l'abdomen. Le pylore et le duodénum sont isolés. Une incision est pratiquée à 1 cm du pylore et les produits sont introduits à l'aide d'une sonde dans le duodénum. Une ligature est effectuée en dessous de l'incision à l'aide de fil chirurgical. L'administration orale est effectuée par gavage à l'aide d'une sonde intragastrique.

Les prélèvements sanguins pour l'analyse des divers paramètres pharmacologiques sont réalisés à l'aorte abdominale respectivement 1 heure après l'administration intraduodénale et 2 heures après l'administration orale.

Ils sont rendus incoagulables par une solution de citrate trisodique à 3,8 p.100 (1 volume d'anticoagulant pour 9 volumes de sang) ou de l'héparine (250 U/ml de sang) pour les mesures de lipoprotéine lipase.

Immédiatement après le prélèvement sanguin, les échantillons sont centrifugés à 2 000 x g pendant 15 minutes à 4 °C pour la préparation du plasma citraté qui est utilisé pour les différents dosages.

Les résultats sont rassemblés dans les tableaux 1 à 6 ; leur analyse statistique a été effectuée selon le test de STUDENT (groupe de comparaison : SP 54).

## TABLEAU 1

## VOIE INTRADUODENALE

## TEMPS de CEPHALINE ACTIVEE

| Produit | Ma | 400 mg/kg | | | |
|---|---|---|---|---|---|
| | | $M \pm ESM$ sec | % (a) | test "t" vs Tem | test "t" vs SP54 |
| TEMOINS | | 20 ± 0,6 | -- | -- | n.s. |
| Exemple 1 | 3346 | 32 ± 1,5 | 60 | p<0,001 | p<0,001 |
| Exemple 2 | 3518 | 32 ± 1,6 | 60 | p<0,001 | p<0,001 |
| Exemple 3 | 3889 | 24 ± 0,5 | 20 | p<0,01 | p<0,01 |
| Exemple 5 | 4066 | 28 ± 0,9 | 40 | p<0,001 | p<0,001 |
| Exemple 6 | 4407 | 29 ± 1,9 | 45 | p<0,01 | p<0,01 |
| Exemple 8 | 4903 | 24 ± 0,7 | 20 | p<0,01 | p<0,05 |
| Exemple 9 | 4921 | 29 ± 0,7 | 45 | p<0,001 | p<0,001 |
| SP 54 | 6600 | 21 ± 0,4 | 5 | n.s. | -- |

(a) : % prolongation

## TABLEAU 2

## VOIE INTRADUODENALE

### ACTIVITE ANTI-Xa (TEMPS DE YIN)

| Produit | Ma | 400 mg/kg | | | |
|---|---|---|---|---|---|
| | | M±ESM sec | % (a) | test "t" vs | |
| | | | | Tem | SP54 |
| TEMOINS | | 33 ± 0,6 | -- | -- | n.s. |
| Exemple 1 | 3346 | 36 ± 0,5 | 9 | p<0,05 | p<0,05 |
| Exemple 2 | 3518 | 36 ± 0,3 | 9 | p<0,05 | p 0,05 |
| Exemple 3 | 3889 | 33 ± 0,3 | 0 | n.s. | n.s. |
| Exemple 5 | 4066 | 38 ±0,0 | 15 | p<0,01 | p 0,05 |
| Exemple 6 | 4407 | 35 ± 1,9 | 6 | n.s. | n.s. |
| Exemple 8 | 4903 | 33 ± 0,7* | 0 | n.s. | n.s. |
| Exemple 9 | 4921 | 37 ±0,4 | 12 | p<0,05 | p 0,05 |
| SP 54 | 6600 | 34 ± 1 | 3 | n.s. | -- |

(a) : % prolongation

## TABLEAU 3

## VOIE INTRADUODENALE

## TEMPS DE LYSE DES EUGLOBULINES

| Produit | Ma | 400 mg/kg | | | |
|---|---|---|---|---|---|
| | | M $\pm$ ESM sec | % (a) | test "t" vs Tem | test "t" vs SP54 |
| TEMOINS | | 134 $\pm$ 6 | -- | -- | n.s. |
| Exemple 1 | 3346 | 102 $\pm$ 4 | 24 | p<0,01 | p<0,05 |
| Exemple 2 | 3518 | 97 $\pm$ 4 | 28 | p<0,001 | p<0,001 |
| Exemple 3 | 3889 | 105 $\pm$ 4 | 22 | p<0,01 | p<0,05 |
| Exemple 5 | 4066 | 93 $\pm$ 4 | 30 | p<0,01 | p<0,01 |
| Exemple 6 | 4407 | 72 $\pm$ 2 | 46 | p<0,001 | p<0,001 |
| Exemple 8 | 4903 | 94 $\pm$ 8 | 30 | p<0,01 | p<0,05 |
| Exemple 9 | 4921 | 99 $\pm$ 5 | 26 | p<0,01 | p<0,01 |
| SP 54 | 6600 | 124 $\pm$ 8 | 7 | n.s. | -- |

(a) : % d'activation

**TABLEAU 4**

**VOIE INTRADUODENALE**

**LIPOPROTEINE LIPASE (L.P.L.)**

| Produit | Ma | 400 mg/kg | | | |
|---|---|---|---|---|---|
| | | M$\pm$ESM * | % (a) | test "t" vs Tem | test "t" vs SP54 |
| TEMOINS | | 3,2 $\pm$ 0,3 | 6 | -- | p<0,001 |
| Exemple 1 | 3346 | 14,1 $\pm$1,6 | 26 | p<0,001 | p<0,01 |
| Exemple 2 | 3518 | 15 $\pm$ 2 | 28 | p<0,001 | p<0,01 |
| Exemple 3 | 3889 | 7,1 $\pm$0,8 | 13 | p<0,001 | n.s. |
| Exemple 5 | 4066 | 15 $\pm$ 0,65 | 28 | p<0,001 | p<0,01 |
| Exemple 6 | 4407 | 3,9 $\pm$0,7 | 7 | n.s. | p<0,01 |
| Exemple 8 | 4903 | 8,7 $\pm$1,1 | 16 | p<0,001 | n.s. |
| Exemple 9 | 4921 | 15,1$\pm$1,5 | 28 | p<0,001 | p<0,01 |
| SP 54 | 6600 | 7,6 $\pm$0,6 | 14 | p<0,001 | -- |

(a) : % de libération

* mol acide oléique/ml/h.

## TABLEAU 5

## VOIE ORALE

## TEMPS DE CEPHALINE ACTIVEE

| Produit | Ma | 400 mg/kg | | | |
|---|---|---|---|---|---|
| | | M ± ESM sec | % (a) | test "t" vs Tem | test "t" vs SP54 |
| TEMOINS | | 20 ± 0,4 | -- | -- | -- |
| Exemple 1 | 3346 | 29 ± 0,5 | 45 | p<0,001 | p<0,001 |
| Exemple 2 | 3518 | 31 ± 0,6 | 55 | p<0,001 | p<0,001 |
| Exemple 3 | 3889 | 21 ± 0,8 | 5 | n.s. | n.s. |
| Exemple 5 | 4066 | 29 ± 0,6 | 45 | p<0,001 | p<0,001 |
| Exemple 6 | 4407 | 21 ± 0,3 | 5 | n.s. | n.s. |
| Exemple 8 | 4903 | 21 ± 0,8 | 5 | n.s. | n.s. |
| Exemple 9 | 4921 | 29 ± 0,6 | 45 | p<0,001 | p<0,001 |
| SP 54 | 6600 | 20 ± 0,4 | 0 | n.s. | -- |

(a) : % prolongation

TABLEAU 6

ACTIVITE PROAGREGANTE VIS-A-VIS ADP

| Produit | Ma | Concentration critique en polysaccharides sulfatés (a) en µg/ml |
|---|---|---|
| Exemple 1 | 3346 | > 125 |
| Exemple 2 | 3518 | 125 |
| Exemple 3 | 3889 | ≥ 125 |
| Exemple 5 | 4066 | > 125 |
| Exemple 6 | 4407 | > 125 |
| Exemple 8 | 4903 | > 125 |
| Exemple 9 | 4921 | > 125 |
| SP 54 | 6600 | 30 |
| Héparine | 19800 | 6 |

(a) Concentration critique = concentration minimale en polysaccharides sulfatés permettant l'obtention d'un phénomène irréversible d'agrégation à l'ADP sur PRP humain.

Les résultats précédents montrent les intéressantes propriétés anti-thrombotiques et hypolipémiantes des produits de l'invention.

En raison de leur bas poids moléculaire, ceux-ci présentent, en effet, par voie orale et surtout intraduodénale, des activités supérieures à celles du SP 54 dont ils sont issus.

Pour chaque voie et chaque produit, ces effets sont d'autant plus nets que la masse moléculaire est plus faible dans la zone revendiquée.

Ainsi, le produit décrit dans l'exemple 1 provoque, à la dose de 400 mg/kg par voie intraduodénale, un allongement de 60 % du temps de céphaline activée (TCA) alors que le SP 54, dans les mêmes conditions, ne manifeste aucune activité significative.

Enfin, à l'inverse du SP 54 et surtout de l'héparine, les produits de l'invention ne potentialisent pratiquement pas l'agrégation in vitro des plaquettes humaines stimulées à l'ADP. Ces résultats laissent donc espérer moins de risques thrombocytopéniants qu'avec les produits de référence.

Le médicament de l'invention peut être présenté sous les formes administrables par les voies orale, parentérale, rectale et locale.

Chaque dose unitaire contient avantageusement de 0,030 g à 0,250 g de principe actif, les doses administrables journellement pouvant varier de 0,030 g à 1,000 g en fonction de la gravité de l'affection traitée et de la voie d'administration.

On donnera ci-après, à titre d'exemple non limitatif, quelques formulations pharmaceutiques du médicament de l'invention.

1 - Comprimés

- dosés à 250 mg de principe actif

| | | |
|---|---|---|
| Principe actif (fraction de l'exemple 1) | 250 | mg |
| Lactose.................................. | 50 | mg |
| Cellulose excipient...................... | 60,1 | mg |
| Magnésium stéarate....................... | 9 | mg |
| Silicium dioxyde colloïdal............... | 0,8 | mg |
| Indigotine laque aluminique.............. | 0,1 | mg |

Pour un comprimé nu terminé à 370 mG.

Pour la forme précédemment citée, on peut réaliser un pelliculage gastrosoluble ou un pelliculage gastrorésistant dont les formules sont les suivantes :

- Pelliculage gastrosoluble

| | |
|---|---|
| Polyméthylacrylate de butyle et diméthylaminoéthyle, granulé.......... | QS |
| Isopropanol pharmaceutique industriel... | QS |
| Acétone.................................. | QS |
| Phtalate de dibutyle..................... | QS |
| Acide silicique.......................... | QS |

- Pelliculage gastrorésistant

| | |
|---|---|
| Polyméthylacrylate anionique, type 1, poudre | QS |
| Isopropanol pharmaceutique industriel... | QS |
| Acétone.................................. | QS |
| Phtalate de dibutyle..................... | QS |

2 - Gélules

| | |
|---|---|
| Principe actif (fraction de l'exemple 6) | 0,075 g |
| Excipient.........q.s.p................. | 1 gélule |

3 - Solutés injectables

| | |
|---|---|
| Principe actif (fraction de l'exemple 5) | 0,100 g |
| Solvant isotonique.....q.s.p............ | 1 ampoule de 2 ml |

4 - Pommade

| | |
|---|---|
| Principe actif (fraction de l'exemple 9) | 0,150 g |
| Excipient........q.s.p.................. | 1 tube de 30 g |

Par leur action sur les différents stades de la coagulation sanguine et de la fibrinolyse, ces médicaments peuvent être utilisés avec profit, en cures prolongées et répétées dans le traitement préventif ou curatif d'accidents thromboemboliques veineux ou artériels dans les cas de phlébites, embolies pulmonaires et autres situations cliniques du même type, angines de poitrine, infarctus du myocarde, artérites chroniques des membres inférieurs, troubles circulatoires superficiels et ulcères des jambes.

Ils peuvent également être utilisés pour prévenir les thromboses dans les circuits extra-corporels (hémodialyses rénales).

En outre, leur action sur la lipoprotéine-lipase (facteur clarifiant) les rend particulièrement utiles dans le traitement des dyslipémies athérogènes.

Enfin, ces médicaments peuvent également être utilisés avec profit dans le traitement de certaines maladies inflammatoires, telles que polyarthrite rhumatoïde, arthroses et ostéoarthrites.

# REVENDICATIONS

1. Sulfates de xylane ayant un degré de sulfatation moyen compris entre 1,5 et 2, un pourcentage en acides uroniques compris entre 0 et 10, un pourcentage en pentoses compris entre 30 et 40, caractérisés par une masse moléculaire moyenne apparente comprise entre 2000 et 5000 Daltons.
2. Procédé de préparation des composés selon la revendication 1 caractérisé en ce que le polysulfate de xylane SP 54 est soumis à un fractionnement.
3. Procédé de préparation des composés selon la revendication 1 caractérisé en ce que le fractionnement est obtenu par ultra-filtration.
4. Médicament ayant notamment des activités antithrombotique et lipolytique, caractérisé en ce qu'il contient à titre de principe actif un composé tel que défini à la revendication 1.
5. Médicament selon la revendication 4 caractérisé en ce qu'il est présenté sous forme appropriée à l'administration orale, parentérale, rectale ou locale associé à des véhicules ou excipients thérapeutiquement acceptables.
6. Médicament selon la revendication 5 caractérisé en ce qu'il est présenté sous forme de doses unitaires contenant chacune de 0,030 g à 1,00 g de principe actif.

## REVENDICATIONS

1. Procédé de préparation de sulfates de xylane ayant un degré de sulfatation moyen compris entre 1,5 et 2, un pourcentage en acides uroniques compris entre 0 et 10, un pourcentage en pentoses compris entre 30 et 40, et une masse moléculaire moyenne apparente comprise entre 2000 et 5000 Daltons.
2. Procédé de préparation des composés selon la revendication 1, caractérisé en ce que le fractionnement est obtenu par ultra-filtration.
3. Procédé de préparation d'un médicament ayant notamment des activités antithrombotique et lipolytique, caractérisé en ce que l'on met sous forme thérapeutiquement acceptable un composé obtenu selon la revendication 1 ou 2.

Office européen des brevets

**RAPPORT DE RECHERCHE EUROPEENNE**

0184480

Numero de la demande

EP 85 40 2157

## DOCUMENTS CONSIDERES COMME PERTINENTS

| Catégorie | Citation du document avec indication, en cas de besoin des parties pertinentes | Revendication concernee | CLASSEMENT DE LA DEMANDE (Int. Cl 4) |
|---|---|---|---|
| Y | FR-A-2 543 145 (SANOFI)<br>* Exemples 10,12-14; pages 18,22 *<br>--- | 1-6 | C 08 B 37/14<br>A 61 K 31/72 |
| Y | JOURNAL OF CHROMATOGRAPHY, vol. 297, 3 août 1984, pages 351-358, Elsevier Science Publishers B.V., Amsterdam, NL; D. MULLER et al.:"High-pressure size-exclusion chromatography of anticoagulant materials"<br>* Page 351, avant-dernier alinéa; page 357, conclusions *<br>----- | 1-6 | |
| | | | DOMAINES TECHNIQUES RECHERCHES (Int. Cl.4) |
| | | | C 08 B<br>A 61 K |

Le present rapport de recherche a ete etabli pour toutes les revendications

| Lieu de la recherche | Date d achèvement de la recherche | Examinateur |
|---|---|---|
| LA HAYE | 24-02-1986 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82